(19) Europäisches Patentamt
European Patent Office
Office européen des brevets

(11) Publication number : 0 586 234 A2

(12) # EUROPEAN PATENT APPLICATION

(21) Application number : **93306889.2**

(22) Date of filing : **01.09.93**

(51) Int. Cl.$^5$ : **A61K 7/48,** A61K 7/08, A61K 7/50, C11D 1/83

(30) Priority : **04.09.92 GB 9218792**

(43) Date of publication of application : **09.03.94 Bulletin 94/10**

(84) Designated Contracting States : **AT BE CH DE DK ES FR GB GR IE IT LI NL PT SE**

(71) Applicant : **UNILEVER PLC**
**Unilever House Blackfriars**
**London EC4P 4BQ (GB)**
(84) **GB IE**

(71) Applicant : **UNILEVER N.V.**
**Weena 455**
**NL-3013 AL Rotterdam (NL)**
(84) **BE CH DE DK ES FR GR IT LI NL PT SE AT**

(72) Inventor : **Hagan, Desmond Bernard**
**35 Hookstone Drive, Little Sutton**
**South Wirral, Cheshire L66 4TE (GB)**
Inventor : **Lyle, Ian Gardner**
**60 Highland Avenue, Aston Park**
**Deeside, Clwyd CH5 1XQ, Wales (GB)**

(74) Representative : **Ford, Michael Frederick et al**
**MEWBURN ELLIS 2 Cursitor Street**
**London EC4A 1BQ (GB)**

(54) **Skin cleansing composition.**

(57) A cosmetic composition suitable for topical application to the human skin or hair, especially for use as a make-up remover, comprising at least one secondary or branched chain nonionic alcohol ethylene oxide condensation surfactant having an ethylene oxide number between 4 and 12, or a secondary or branched chain nonionic alcohol ethylene oxide/propylene oxide condensation surfactant having a propylene oxide condensation number between 1 and 6 and an ethylene oxide condensation number of between 4 and 20, the nonionic surfactant having an average HLB value of 10-14, together with at least one low-irritant anionic surfactant which is preferably selected from the group comprising monoalkyl phosphates and/or dialkyl phosphates, alkyl ether phosphates, alkyl ether sulphates, alkyl ether carboxylates, N-acyl sarcosinates N-acyl glutamates, acyl isethionates, acyl methyl taurates, acyl lactylates, alkylamphocarboxyglycinates, alkyl sulphosuccinates and mixtures thereof.

EP 0 586 234 A2

This invention relates to a cleansing composition for topical application to parts of the body, such as the skin and hair. In particular, it relates to a skin cleansing composition that is sufficiently mild that it is suitable for topical application to the human skin, and yet is sufficiently efficacious that it is capable of removing greasy make-up.

EP-A-0324575 (Kao) describes a make-up removal composition comprising a branched chain nonionic surfactant having an HLB value of between 3 and 8, together with an anionic surfactant selected from monoalkyl phosphates or dialkyl phosphates, N-acyl glutamates, isethioniates and sulphosuccinates. However, this reference is concerned only with those branched chain nonionic surfactants which have an HLB value of from 3 to 8. The document furthermore gives clear-teaching that similar nonionics with an HLB value outside this range cannot be used in the subject compositions because, so the document says, they lack sufficiently high detergency to remove make-up or other hard, greasy deposits.

Surprisingly, we have found that, contrary to the teaching of this reference, foamable, low-irritant makeup removal compositions otherwise similar to those described in EP-A-0324575 can be made which comprise a nonionic surfactant having an HLB value not in the range 3-8 but in the range 10-14, and having a carefully selected content of ethylene oxide/propylene oxide units. These compositions do in fact exhibit a sufficient degree of detergency to act as efficient make-up removers. Such compositions have also been found to exhibit a high degree of foaming, and a low degree of irritancy.

Thus, according to the invention, there is provided a cosmetic composition suitable for topical application to the human skin or hair, comprising at least one secondary or branched chain nonionic alcohol ethylene oxide condensation surfactant having an ethylene oxide number between 4 and 12, or a secondary or branched chain nonionic alcohol ethylene oxide/propylene oxide condensation surfactant having a propylene oxide condensation number between 1 and 6 and an ethylene oxide condensation number between 4 and 20, the nonionic surfactant having on average HLB value of from about 10 to about 14, especially from about 10.5 to about 14, together with at least one low-irritant anionic surfactant which is preferably selected from monoalkyl phosphates and/or dialkyl phosphates, alkyl ether phosphates, alkyl ether sulphates, alkyl ether carboxylates, N-acyl sarcosinates, N-acyl glutamates, acyl isethionates, acyl methyl taurates, acyl lactylates, alkylamphocarboxyglycinates, alkyl sulphosuccinates and mixtures thereof. Preferably, the nonionic surfactant in the composition has an average HLB value of from about 10 or 10.5 to about 12.5.

The present invention, and in particular preferred features and embodiments thereof, will now be described in detail.

The nonionic surfactant in the composition may be present at a concentration of from about 2 to about 20% by weight, preferably from about 5 to about 15% by weight, more preferably from about 8 to about 12% by weight of the composition.

The anionic surfactant in the composition may be present at a concentration of from about 8 to about 58% by weight, preferably from about 10 to about 35% by weight, more preferably from about 15 to about 25% by weight of the composition.

Preferably, the total amount of surfactant in the composition is in the range of from about 10 to about 60% by weight, more preferably from about 25 to about 40% by weight of the composition.

The secondary or branched chain nonionic alcohol ethylene oxide or ethylene oxide/propylene oxide condensation surfactant is preferably an alkyl or alkenyl nonionic alcohol condensate.

Preferred examples of the hydrocarbon group include secondary or branched chain alkyl groups each having from about 4 to about 30 carbon atoms. Particular preferred examples of the hydrocarbon group are selected from among 2-ethylhexyl, 2-ethyloctyl, 2-ethyldecyl, 2-ethyldodecyl, 2-butyloctyl, 2-butyldecyl, 2-butyldodecyl, 2-butyltetradecyl, 2-hexyldecyl, 2-hexyldodecyl, 2-hexyltetradecyl, 2-hexylhexadecyl, 2-octyldecyl, 2-octyldodecyl, 2-octyltetradecyl, 2-octylhexadecyl, 2-octyloctyldecyl, 2-decyltetradecyl, 2-heptylundecyl and methyl-branched isostearyl groups. Among these examples, branched chain or secondary alkyl groups each having 10 to 14 carbon atoms, and 2-hexyldecyl, 2-ethylhexyl, 2-octyldodecyl and 2-heptylundecyl groups are especially preferred.

Suitable nonionic ethylene oxide condensation surfactants for use in the invention are those having an EO number of from about 4 to about 12, preferably from about 5 to about 9, i.e. that number of ethylene oxide residues per molecule, and which have the above-defined average HLB value.

Suitable nonionic ethylene oxide/propylene oxide mixed condensation surfactants for use in the invention are those having a propylene oxide condensation number of from about 1 to about 6, preferably from about 1 to about 4, and an ethylene oxide condensation number of from about 4 to about 20, preferably from about 4 to about 10, and which have the above-defined average HLB value.

Thus, preferred ethylene oxide/propylene oxide nonionic surfactants for use in the invention have the general formula (I):

$$R_1-O(CH_2\underset{\displaystyle CH_3}{\underset{|}{C}}HO)_x-(CH_2CH_2O)_y-H \qquad (I)$$

wherein $R_1$ is a secondary or branched chain alkyl hydrocarbon group having from 4 to 30 carbon atoms, x is an integer from 1 to 6 and y is an integer from 4 to 20, and having an average HLB value in the range 10-14.

Preferred examples of nonionic surfactants for use in the invention are secondary alcohol ethoxylates, in particular C12-C14 secondary alcohol ethoxylates, having 5-7 ethylene oxide groups. These preferably have an HLB value of 10-12.5.

The cosmetic compositions of the invention comprise, in addition to the nonionic surfactant, at least one anionic surfactant which is of the class well recognised in the art as being low-irritant anionic surfactants.

Suitable anionic surfactants for use in the invention are selected from the following: monoalkyl phosphates and/or dialkyl phosphates, alkyl ether phosphates, alkyl ether sulphates, alkyl ether carboxylates, N-acyl sarcosinates, N-acyl glutamates, acyl isethionates, acyl methyl taurates, acyl lactylates, alkylamphocarboxyglycinates and alkyl sulphosuccinates, especially their sodium, magnesium, ammonium and mono-, di- and triethanolamine salts. The alkyl and acyl groups generally contain from 8 to 18 carbon atoms and may be unsaturated.

In the cases where these anionics contain ether groups, there are present preferably from 1 to 10, especially 1, 2 or 3 (or possibly 4) ethylene oxide and/or propylene oxide residues per molecule.

It is to be understood that the above named examples of low-irritant anionic surfactants for use in the invention are not intended to exclude other low-irritant anionics which may be suitable for use in the invention by virtue of their sufficiently low irritancy to human skin or hair.

Preferred examples of low-irritant anionic surfactants for use in the invention are sodium mono- or dialkyl phosphates, acyl methyl taurates, and acyl lactylates. If the anionic surfactant according to the invention comprises both monoand dialkyl phosphates, it is preferred that the ratio of monoalkyl phosphate surfactant to dialkyl phosphate surfactant is in the range 1:0 to 1:1, preferably 1:0 to 3:2.

In addition to the above low-irritant anionic surfactant, the cosmetic composition of the present invention may further contain a conventional surfactant such as fatty acid soap or alkyl sulphate, provided that the low irritancy and high detergency properties of the composition of the invention are not adversely affected.

Further, for the purpose of enhancing the ability to foam, the composition of the present invention may further contain an anionic, amphoteric or nonionic surfactant in addition to the above essential components, provided that the effectiveness and mildness of the composition is not affected adversely.

The cosmetic compositions of the invention may further contain a thickener such as an anionic or nonionic polymer, or other conventional additives, for example stabilizers, perfumes or dyestuffs. A preferred thickener for compositions according to the inventions is a cationic polymer, in particular the cationic polymer Polymer JR, and the JAGUAR range of polymers.

As described above, according to the present invention, a cleansing foamable composition for application to the face or body which exhibits sufficient efficacy to remove makeup smear and yet excellent mildness, can be provided by combining a particular secondary or branched chain nonionic surfactant having the specified HLB value with at least one low-irritant anionic surfactant preferably selected from the exemplary group of low-irritant anionic surfactants mentioned hereinabove. Skin cleansing compositions according to the invention are additionally thought to impart a degree of moisturisation to the skin.

Further optional components of compositions according to the invention may include for example colouring agents, preservatives, pearlescing agents, humectants such as sorbitol and glycerol, perfumes and buffering agents.

The present invention will be described in more detail by referring to the following non-limiting examples:

## Example 1

Skin cleansing compositions of the formulations given in Table 1 below were prepared and tested according to the following test protocol for removing lipstick from white plastics expanded polyethylene discs.

Table 1

Formulation % w/w

| COMPONENT | | 1 | 2 | 3 | 4 | 5 | 6 | 7 | 8 | 9 | 10 | 11 | 12 |
|---|---|---|---|---|---|---|---|---|---|---|---|---|---|
| SOFTANOL 70 | (a) | 10 | | 10 | 10 | | | | | | | | |
| SOFTANOL 50 | (b) | | 10 | | | 10 | | | | 10 | 10 | 10 | 10 |
| EMALEX 1605 | (c) | | | | | | 10 | 10 | 10 | | | | |
| TEA MAP | (d) | 20 | 20 | 20 | | | 20 | 20 | | | | | |
| TEA LAURATE | (e) | | | 5 | | | | 5 | | | | | |
| AMT | (f) | | | | 20 | 20 | | | | | | | |
| N-LAUROYL GLUTAMATE | | | | | | | | | 20 | | | | |
| SODIUM LAUROYL LACTYLATE (g) | | | | | | | | | | 20 | 20 | | |
| PATIONIC 122A | (h) | | | | | | | | | | | 20 | |
| PATIONIC 138C | (i) | | | | | | | | | | | | 20 |
| TEGOBETAINE C | (j) | | | | | | | | | 10 | | | |
| ANON GLM-S | (k) | | | | | | | | | | 10 | 5 | 5 |
| DISTILLED WATER | | to 100 | --- | --- | --- | --- | --- | --- | --- | --- | --- | --- | to 100 |

(a) SOFTANOL 70 is a $C_{12}/_{14}$ secondary alcohol ethoxylate, 7 ethylene oxide groups, HLB 12.1, available from BP Chemicals.

(b) SOFTANOL 50 is a $C_{12}/_{14}$ secondary alcohol ethoxylate, 5 ethylene oxide groups, HLB 10.5, available from BP Chemicals.

(c) EMALEX 1605 is a polyethylene oxide (5 units) hexyl decyl ether, HLB 7.0, avialable from Nihon Emulsion Co.

(d) TEA MAP is Hoechst HN98 (monolauryl phosphoric acid, adjusted with triethanolamine to pH 6).

(e) TEA LAURATE is triethanolamine laurate, a foam-booster surfactant.

(f) AMT is Diapon K (cocyl N-methyl sodium taurate), available from Nippon Oil & Fats.

(g) ex Croda.

(h) Sodium C10/12 acyl lactylate, available from Rita Corporation.

(i) Sodium C12/14 acyl lactylate, available from Rita Corporation.

(j) Cocoamidopropyl betaine, available from Goldschmidt.

(k) A mixture of cocoamphoacetate/N-coconut fatty acid amidoethyl-N-2-hydroxylethyl glycinate and N-coconut fatty acid amidoethyl-N-2-hydroxyethyl-N-carboxymethyl glycinate, available from Nippon Oil & Fats.

Examples 6-8 are outside the scope of the invention and are provided for comparison only.

Experimental protocol

The skin cleansing compositions according to the invention were evaluated in comparative tests with respect to their makeup removal efficacy for lipstick.

Before use, each make up remover sample was diluted with two parts of water; the purpose of this was to provide a surfactant concentration in the solution as used of around 10%, a typical surfactant concentration in topical use.

The lipstick was applied to a white plastics disc in a 3 cm x 2 cm rectangle. Before lipstick application, the exact colour of the disc was measured using a Minolta Chroma meter CR-100 to give a set of baseline readings (A). Each recorded result was the average of 6 individual measurements.

The lipstick was then applied to the plastics disc in a standardised way to ensure that approximately equal weights of make up were applied. After application, a second colour measurement was made (reading B).

Thereafter, a standard amount of test product (0.2 ml after dilution) was applied to the plastics disc, and rubbed lightly into the lipstick for a period of 20 seconds using light hand pressure from a gloved hand. Afterwards the mixture was immediately rinsed off with running tap water at a fixed flow rate and temperature (35°C) for 20 seconds. The discs were then allowed to dry, and final colour measurements made (reading C).

The percentage removal of make up was calculated as;

$$\frac{B - C}{B - A} \times 100\ \%$$

Results

| Formulation | % lipstick removal |
|---|---|
| 1 | 79.5 % |
| 2 | 83.3 % |
| 3 | 87.3 % |
| 4 | 78.6 % |
| 5 | 79.8 % |
| 6 | 58.4 % |
| 7 | 62.7 % |
| 8 | 36.0 % |
| 9 | 72.3 % |
| 10 | 71.2 % |
| 11 | 81.3 % |
| 12 | 70.6 % |

These results demonstrate effective removal of make-up, in particular greasy make-up such as lipstick, for compositions according to the invention, which are significantly superior to comparative compositions 6-8 which are not within the scope of the invention.

Foam generation

An important feature of compositions according to the invention is their ability to produce high levels of foam. Formulations 1-12 were subjected to a foam generation test according to the Ross-Miles protocol, conducted on solutions containing 1 % of each of formulations 1-12.

Protocol

According to the Ross Miles method, 200 ml of the test solution is placed in the bottom of a tall glass column of 50 mm diameter and at 25°C, and 200 ml of the same solution is dropped from a reservoir at an initial height of 755mm above the liquid surface into the cylinder. The solution in the reservoir is allowed to fall dropwise over a period of 1 minute. Immediately the 1 minute is complete and the full 200 ml is added, the foam height is measured.

Results

| Formulation | Foam Height (mm) |
|---|---|
| 1 | 120 |
| 2 | 116 |
| 3 | 148 |
| 4 | 115 |
| 5 | 116 |
| 6 | 90 |
| 7 | 130 |
| 8 | 45 |
| 9 | 145 |
| 10 | 143 |
| 11 | 136 |
| 12 | 151 |

The results demonstrate remarkably good foaming properties for compositions according to the invention.

## Claims

1. A cosmetic composition suitable for topical application to the human skin or hair, comprising at least one secondary or branched chain nonionic alcohol ethylene oxide condensation surfactant having an ethylene oxide condensation number between 4 and 12, or a secondary or branched chain nonionic alcohol ethylene oxide/propylene oxide condensation surfactant having a propylene oxide condensation number between 1 and 6 and an ethylene oxide condensation number between 4 and 20, the nonionic surfactant having an average HLB value of from 10 to 14, together with at least one low-irritant anionic surfactant.

2. A composition according to claim 1, wherein the anionic surfactant is selected from the group which includes monoalkyl phosphates and/or dialkyl phosphates, alkyl ether phosphates, alkyl ether sulphates, alkyl ether carboxylates, N-acyl sarcosinates, N-acyl glutamates, acyl isethionates, acyl methyl taurates, acyl lactylates, alkylamphocarboxyglycinates, alkyl sulphosuccinates and mixtures thereof.

3. A composition according to claim 1 or claim 2, wherein the nonionic surfactant has an average HLB value of from 10 to 12.5.

4. A composition according to any one of claims 1 to 3, wherein the nonionic surfactant is present in an amount of from 2 to 20% by weight of the composition.

5. A composition according to any preceding claim, wherein the anionic surfactant is present in an amount of from 8 to 58% by weight of the composition.

6. A composition according to any preceding claim, wherein the total amount of surfactant present in the composition is from 10 to 60% by weight thereof.

7. A composition according to any preceding claim, wherein the secondary or branched chain nonionic alcohol ethylene oxide or ethylene oxide/propylene oxide condensation surfactant is an alkyl or alkenyl nonionic alcohol condensate.

8. A composition according to claim 7, wherein the nonionic surfactant is an alkyl nonionic alcohol condensate and the alkyl group is selected from secondary or branched chain alkyl groups each having from 4 to 30 carbon atoms.

**9.** A composition according to any preceding claim, wherein the nonionic surfactant is selected from C12 to C14 secondary alcohol ethoxylates having from 5 to 7 ethylene oxide residues per molecule and having an HLB value of from 10 to 12.5.

**10.** A composition according to any one of claims 1 to 8, wherein the nonionic surfactant has the general formula (I):

$$R_1-O(CH_2\underset{\displaystyle CH_3}{\underset{|}{C}}HO)_x-(CH_2CH_2O)_y-H \qquad (I)$$

wherein $R_1$ is a secondary or branched chain alkyl hydrocarbon group having from 4 to 30 carbon atoms, x is an integer from 1 to 6 and y is an integer from 4 to 20, and having an average HLB value in the range 10-14.

**11.** A composition according to any preceding claim, wherein the low-irritant anionic surfactant comprises a mixture of mono- and dialkyl phosphates, the ratio of monoalkyl phosphates surfactant to dialkyl phosphate surfactant being in the range 1:0 to 1:1.

**12.** A composition according to any preceding claim, further including one or more additional surfactants selected from anionic, amphoteric or nonionic surfactants.

**13.** A composition according to any preceding claim, further including a thickener.

**14.** A method of removing make-up from human skin or hair, comprising topically applying thereto a composition according to any one of claims 1 to 13.